# DEMANDE DE BREVET EUROPEEN

(11) **EP 4 778 547 A1**
(43) Date de publication de la demande: **22.07.2026**
(21) Numéro de dépôt: 25152661.2
(22) Date de dépôt: 17.01.2025
(51) Int. Cl.: A61M 5/00, A61M 5/145, A61M 5/168

(54) **AUTOMATE MOBILE POUR PREPARER ET ADMINISTRER UNE DOSE RADIOPHARMACEUTIQUE À UN PATIENT ET METHODES DE MISE EN OEUVRE DE L'AUTOMATE**

(71) Demandeur: Trasis S.A., 4430 Ans (BE)
(72) Inventeur: MORELLE, Jean-Luc, 4000 LIEGE (BE); PHILIPPART, Gauthier, 4280 AVIN (BE); MARECHAL, Adrien, 4606 DALHEM (BE); PIRONT, Guillaume, 4452 WIHOGNE (BE)
(74) Mandataire: AWA Benelux

(57) **Abrégé**

La présente invention se rapporte à un appareil mobile (1) pour la préparation et l'injection ou la perfusion automatique à la demande d'une dose de produit radiopharmaceutique à une pluralité de patients, dans lequel une trousse (32, 100) comprend une seringue commandée (101) pour prélever le produit radiopharmaceutique après percement au moyen d'un système motorisé d'un septum du flacon de produit radiopharmaceutique (31, 107) et une vanne trois-voies (103) qui permet successivement de prélever le produit radiopharmaceutique dans la seringue (101), de le mélanger à une quantité de saline (33, 109) et de l'injecter à un patient selon trois chemins de fluide respectifs (106, 108, 110).

## Description

### Domaine de l'invention

La présente invention concerne un automate mobile conçu pour préparer et administrer (injecter/perfuser) une dose de produit radiopharmaceutique à un patient. L'invention concerne également une ou plusieurs méthodes pour la mise en oeuvre de l'appareil.

Plus précisément, l'invention concerne un automate mobile de préparation et d'injection directe au patient de traceurs de tomographie à émission de positrons (TEP), respectueux des normes radiopharmaceutiques en vigueur, assurant la radioprotection du personnel hospitalier et la traçabilité de l'ensemble des opérations au TEP.

### Arrière-plan technologique et état de la technique

Principalement utilisés en imagerie médicale ou en médecine nucléaire, les produits radioactifs doivent être dosés avec précision avant administration à un patient et sans exposer le personnel soignant.

Actuellement, il est connu que l'administration d'un produit radioactif à un patient requiert idéalement des dispositifs faciles à utiliser, largement automatisés et mobiles.

Un dispositif d'injection mobile conçu pour administrer des produits radiopharmaceutiques en milieu clinique doit ainsi respecter un certain nombre de spécifications essentielles.

Tout d'abord, il doit être compact et léger, ce qui facilite son transport et sa maniabilité dans les locaux cliniques. Idéalement, il devrait être monté sur des roues, motorisées ou non, pour pouvoir être déplacé dans les hôpitaux ou les cliniques.

En outre, le mécanisme de préparation et d'administration de dose doit être très précis, pour pouvoir préparer/injecter des doses exactes avec une vitesse et un volume d'injection contrôlés.

Plus précisément, la préparation consiste à fractionner un flacon multidose en mono-doses à destination du patient de manière ultraprécise, chaque monodose prélevée étant alors mesurée de manière ultraprécise. L'injection consiste à injecter la dose préparée dans la seringue d'une trousse dans les veines des patients avec un débit régulé et un contrôle de la pression d'injection. Le dispositif permet aussi idéalement un usage secondaire, une injection de dose préparée dans une seringue blindée externe, par exemple déposée sur la tablette de l'injecteur, pour ensuite être injectée manuellement au patient. La radioprotection requise consiste à protéger les utilisateurs de rayonnements gamma émis par la solution multidose se trouvant dans le flacon et véhiculée dans les tubulures du système. Enfin, la traçabilité consiste à récupérer de manière automatisée/semi-automatisée/par encodage de l'utilisateur les données à tracer comme la dose injectée au patient, les données du patient, les données des consommables (par lecture RF-tag, numéro de lot, volumes, etc.), etc.

La compatibilité de l'appareil avec une large gamme de produits radiopharmaceutiques couramment utilisés dans les procédures diagnostiques et thérapeutiques, y compris la capacité à manipuler différentes formulations et concentrations, est essentielle.

De plus, la protection contre les rayonnements est primordiale afin de protéger les opérateurs, le personnel infirmier et les patients d'une exposition inutile, voire même dangereuse. Des dispositifs de sécurité intégrés, tels que des verrouillages et un bouton d'arrêt, par exemple qui coupe l'alimentation du pousse-seringue, sont nécessaires pour éviter les injections ou les expositions accidentelles.

L'interface de contrôle doit être conviviale pour l'opérateur et comporter un écran tactile ou un clavier pour la saisie des informations relatives au patient, aux tests et autres protocoles de traitement à effectuer, ainsi qu'un affichage clair de l'état de chaque injection et des alertes.

De préférence, deux sources d'alimentation garantissent un fonctionnement ininterrompu, même en cas de coupure de courant ou lors du déplacement de l'appareil : tout d'abord une source DC, sous forme de batterie, qui est la source principale d'alimentation du dispositif et une source AC, sous forme d'un branchement sur secteur, utilisé en cas de batterie faible et pour recharger la batterie. Cette solution permet un usage ininterrompu du dispositif.

Il faut aussi prévoir des options de connectivité, telles que l'intégration aux réseaux hospitaliers, ainsi que le Wi-Fi, le Bluetooth et/ou des ports USB pour le transfert de données et les mises à jour, afin d'améliorer les fonctionnalités de l'appareil.

Ces fonctionnalités sont liées à la traçabilité et à la facilité d'utilisation de l'appareil de par la gestion de la liste des patients qui est effectuée dans un logiciel dédié de l'hôpital et l'injecteur peut s'interfacer avec celui-ci pour récupérer facilement cette liste de patients ainsi que toute une série d'informations comme la dose à préparer et à injecter aux différents patients de la journée. La communication va également dans l'autre sens. Par exemple, une fois la dose injectée au patient, le dispositif renvoie les données d'injection (dose exacte injectée avec l'heure d'injection, lot du traceur injecté, etc.) au logiciel de gestion de l'hôpital soit via une passerelle, soit via USB.

Des capacités de surveillance et de diagnostic à distance permettront avantageusement aux professionnels de la santé de superviser les injections à distance et de gérer les problèmes de dépannage ou de maintenance à distance.

Des caractéristiques de nettoyage et d'entretien faciles, telles que des surfaces aisément nettoyables et des composants accessibles, permettent de garantir la stérilité, d'éviter la contamination croisée entre les patients et de faciliter l'entretien de routine.

Le respect des normes réglementaires, telles que les réglementations de la FDA ou le marquage CE, garantit bien sûr la sécurité et l'efficacité de l'administration des produits radiopharmaceutiques.

Il est aussi nécessaire de disposer de capacités d'établissement de rapports, y compris d'enregistrements détaillés des séances d'injection et de stockage en mémoire à des fins de conformité, d'audit et d'assurance de la qualité.

Enfin, des supports de formation complets et des services d'assistance technique sont essentiels pour les opérateurs et le personnel de santé.

Le document US 9,114,203 B2 divulgue des assemblages de blindage pour des systèmes qui génèrent et perfusent des produits radiopharmaceutiques. Les ensembles de blindage comprennent une paroi latérale qui définit plusieurs compartiments et constitue une barrière contre les rayonnements pour les compartiments, ainsi qu'un passage dimensionné pour permettre l'acheminement d'une ligne d'éluat à partir d'un générateur de radioisotopes. Un premier compartiment est destiné à contenir le générateur de radioisotopes. Un second compartiment est destiné à contenir une partie d'un circuit de perfusion qui peut être une extension de la ligne d'éluat et peut inclure une ligne de déchets et une ligne patient.

Le document US 9,607,722 B2 divulgue des méthodes de mise en place, d'entretien et d'exploitation d'un système de perfusion radiopharmaceutique, qui comprend un générateur de radioisotopes, et dont la mise en oeuvre est facilitée par un ordinateur du système. L'ordinateur comprend des instructions préprogrammées et une interface informatique permettant d'interagir avec un utilisateur du système, par exemple pour suivre les volumes contenant l'éluant et/ou l'éluat, et/ou pour suivre le temps écoulé depuis la fin d'une élution effectuée par le système, et/ou pour calculer un ou plusieurs paramètres du système et/ou de l'injection pour le contrôle de la qualité, et/ou pour effectuer des purges du système, et/ou pour faciliter une imagerie diagnostique.

Le document US 9,750,953 B2 divulgue des systèmes et des méthodes d'administration d'un fluide médical. Le système comprend une voie d'écoulement de fluide, un dispositif d'administration de fluide adapté pour fournir le fluide médical à travers la voie d'écoulement de fluide et un contrôleur en communication avec le dispositif d'administration de fluide. La méthode consiste à utiliser le système pour déterminer un débit souhaité du fluide médical à une extrémité distale de la voie d'écoulement du fluide sur la base d'au moins un profil d'écoulement souhaité du fluide médical à l'extrémité distale. La méthode consiste également à lancer une opération d'administration de fluide en fournissant le fluide médical par le biais du circuit d'écoulement en fonction des paramètres d'administration de fluide fournis au dispositif d'administration de fluide par le contrôleur. Le contrôleur peut recevoir des informations sur l'opération d'administration du fluide et exécuter une fonction de contrôle pour ajuster les paramètres d'administration du fluide sur la base des informations reçues.

Le document US 6,870,175 B2 divulgue une méthode et un appareil de distribution d'un produit radiopharmaceutique dans lesquels : une source de fluide de rinçage est connectée à un premier port d'un ensemble de distribution de fluide ; une unité de pressurisation d'un système d'injecteur motorisé (comprenant un injecteur motorisé et l'unité de pressurisation) est connectée à un deuxième port de l'ensemble de distribution de fluide ; l'air est purgé de l'ensemble de distribution de fluide ; et, après avoir purgé l'air de l'ensemble de distribution de fluide, un troisième port de l'ensemble de distribution de fluide est connecté à une source de produit radiopharmaceutique. Un système de valve est inclus pour contrôler le flux de fluide. Une seringue est reliée à un injecteur motorisé. Un bouclier radioactif entoure la seringue pendant son fonctionnement afin de protéger le personnel des effets néfastes du produit. Un calibrateur de dose mesure la radioactivité dans la seringue.

Le document US 9,627,097 B2 divulgue des systèmes, des appareils et des méthodes grâce auxquels un système d'injection automatise un processus d'injection d'une dose individuelle à partir d'une dose multiple d'un produit radiotraceur. Dans certains cas, le système d'injection comprend un premier système de calibrage de dose qui reçoit un flacon multidose d'un radiotraceur, un second système de calibrage de dose, une pompe d'injection et une aiguille intraveineuse. Dans certains cas, le premier système de calibrage de dose et le flacon multidose ont une forme intégrée. Dans certains modes de réalisation, le premier système de calibrage de dose comprend un bras pneumatique qui reçoit le flacon multidose.

Le document WO 2024/061619 A1 divulgue un dispositif pour l'accouplement réversible d'une seringue à un actionneur, comprenant un corps interne d'axe longitudinal et un manchon externe apte à se déplacer par rapport au corps interne selon l'axe longitudinal, le corps interne comprenant un axe coulissant terminé par un plateau de reprise de jeu maintenu longitudinalement à l'intérieur du corps interne en une position de repos par un ressort précontraint ainsi qu'au moins une paire de crochets disposés longitudinalement à l'intérieur du corps interne, montés pivotants et rétractables deux à deux autour de deux axes respectifs perpendiculaires à l'axe longitudinal, une partie externe des crochets se trouvant en vis-à-vis d'une partie interne du manchon, caractérisé en ce que les crochets d'une même paire possèdent chacun une zone d'accrochage à une extrémité proximale du plateau de reprise de jeu et sont connectés l'un à l'autre à une extrémité distale par un ressort de rappel, la partie distale des crochets étant pourvue, vers l'extérieur, d'un bossage apte à s'insérer dans une zone creuse d'une première profondeur du profil interne du manchon dans une position de repos du dispositif d'accouplement où la zone d'accrochage des crochets est en position de contact de blocage du bouton du poussoir, les crochets étant rapprochés, le coulissement relatif du manchon par rapport au corps interne, dans lequel le manchon s'écarte de ladite extrémité distale des crochets, provoquant l'ouverture des crochets par pivotement autour de leurs axes de pivotement respectifs, du fait que le bossage de chaque crochet entre dans une zone creuse d'une seconde profondeur du profil interne du manchon, la zone creuse d'une seconde profondeur étant adjacente à la zone creuse d'une première profondeur, la première profondeur étant supérieure à la seconde profondeur.

### Objectifs de l'invention

La présente invention a pour but de mettre en oeuvre au moins une grande partie des spécifications énoncées ci-dessus tout en résolvant les problèmes de l'état de la technique.

L'invention vise plus précisément à la mise au point d'un injecteur mobile et automatisé pour la préparation et l'injection ou la perfusion de produits radiopharmaceutiques directement aux patients.

En particulier, elle vise à fournir un équipement et ses consommables, l'ensemble permettant de prélever, mesurer et administrer en toute sécurité une dose prescrite à plusieurs patients sur une journée.

### Principaux éléments caractéristiques de l'invention

Un premier aspect de la présente invention se rapporte à un appareil mobile pour la préparation et l'injection ou la perfusion automatique à la demande d'une dose de produit radiopharmaceutique à une pluralité de patients, comprenant une pluralité de compartiments au moins partiellement blindés :
- un premier compartiment pour accueillir une trousse de tubulures permettant de préparer avec précision via plusieurs chemins de fluide une dose de produit radiopharmaceutique dilué avec de la saline directement injectable à un patient ;
- un deuxième compartiment pour accueillir un flacon multidose de produit radiopharmaceutique ;
- un troisième compartiment pour accueillir un conteneur de saline et la connexion à une ligne patient ;
ladite trousse, le conteneur de saline, et ledit flacon blindé de produit radiopharmaceutique étant des consommables configurés pour être remplacés après traitement d'un ou plusieurs patients ;
ladite trousse comprenant une seringue commandée pour prélever le produit radiopharmaceutique après percement au moyen d'un système motorisé d'un septum du flacon de produit radiopharmaceutique et une vanne trois-voies qui permet successivement de prélever le produit radiopharmaceutique dans la seringue, de le mélanger à une quantité de saline et de l'injecter à un patient selon trois chemins de fluide successifs ; ainsi que
- des moyens de préparation et d'administration de fluide comprenant un actionneur de précision de la seringue commandée et un actionneur de la vanne trois-voies ;
- un activimètre disposé à proximité de la seringue commandée pour valider avant injection la dose préparée ;
- un contrôleur en communication avec les moyens de préparation et d'administration de fluide configuré pour recevoir d'un utilisateur des informations de préparation de dose pour un patient et pour communiquer des instructions correspondantes aux moyens de préparation et d'administration de fluide ;
- une interface de communication entre ledit contrôleur et un utilisateur, capable, via un menu d'instructions, de permettre à l'utilisateur de lancer une procédure de préparation et d'injection de dose, des tests, des calibrations et un accès à des données en mémoire, par exemple relatives à l'historique des injections de dose ; et
- des moyens de stockage et d'impression des données.

Selon des formes d'exécution préférées de l'invention, l'appareil mobile comporte en outre au moins une des caractéristiques suivantes ou une combinaison appropriée de plusieurs d'entre elles :
- l'appareil est un automate mobile, par exemple un chariot mobile monté sur une pluralité de roulettes, déplaçable et guidable au moyen de poignées disposées au moins sur une face avant et muni d'un système de freinage, de préférence grâce à des roulettes autofreinées, ou d'une assistance motorisée des roulettes ;
- lesdites roulettes sont au nombre de quatre, à raison d'une à chaque coin de l'appareil, deux roulettes fixes au niveau d'une face avant, côté utilisateur, et deux roulettes orientables au niveau d'une face arrière, côté patient, de l'appareil ;
- l'interface de communication est une interface utilisateur à écran tactile et disposée sur une surface supérieure de l'appareil ;
- l'appareil est muni sur une face essentiellement verticale, côté patient, d'un premier accès par une première porte blindée au premier compartiment destiné à contenir la trousse de tubulures et d'un second accès par une seconde porte blindée au deuxième compartiment destiné à contenir le flacon multidose de produit radiopharmaceutique ;
- le déverrouillage et/ou l'entrebaillement des première et seconde portes blindées est commandable au moins une fois par jour par l'opérateur à partir de l'interface de communication ;
- l'appareil comprend un capteur de force pour détecter la présence d'une poche de saline et le bon déroulement des mouvements de fluide dans les consommables dont le flacon multidose, la trousse, le conteneur de saline et la ligne patient ;
- l'appareil comporte pour l'échantillonnage et l'injection du produit radiopharmaceutique :
   ∘ un actionneur de percement motorisé pour percer le flacon contenant le produit radiopharmaceutique en manipulant l'aiguille de la trousse ;
   ∘ un pousse-seringue motorisé pour manipuler la seringue de la trousse, en vue de recueillir une dose ultraprécise et de l'injecter ensuite au patient en dilution avec du sérum physiologique ;
- l'appareil comprend des moyens pour une gestion d'ouverture des portes effectuée via l'actionneur de percement pour s'assurer que l'aiguille est bien sortie du flacon lorsqu'on ouvre la porte du flacon et des moyens pour assurer que l'aiguille se positionne dans un logement inaccessible aux doigts de l'utilisateur.

Un second aspect de la présente invention se rapporte à un procédé de mise en oeuvre d'un appareil mobile pour la préparation de doses de produit radiopharmaceutique destinées à l'injection ou la perfusion automatique à la demande à des patients, généralement répartie sur une journée comme décrit ci-dessus, caractérisé par les étapes suivantes :
- mettre l'appareil sous tension au début de la journée ;
- préparer l'appareil pour la journée, à savoir :
   ∘ enlever les consommables dont le flacon multidose, la trousse, le conteneur de saline et la ligne patient, s'ils sont toujours présents depuis la veille, après ouverture de la première porte blindée et de la seconde porte blindée, le flacon étant toujours enlevé avant la trousse pour réduire les risques d'exposition radioactive ;
   ∘ effectuer le test journalier de vérification, en particulier de l'activimètre en utilisant une source de référence ;
   ∘ mettre en place dans l'appareil via l'ouverture des portes précitées de nouveaux consommables, à savoir au moins respectivement un flacon multidose de produit radiopharmaceutique, à destination de plusieurs patients, ainsi qu'une trousse de tubulures et un conteneur de saline ;
   ∘ mettre en place une ligne patient pour chaque nouveau patient ;
- démarrer la procédure automatique de préparation de dose ;
- laisser les tubulures se remplir par la poche de saline et mesurer l'activité dans le flacon en connaissant de manière précise le volume prélevé ;
- détecter la ligne patient ;
- sélectionner, corriger et/ou encoder sur l'interface de communication un patient déterminé et une dose prescrite ;
- effectuer la préparation de la dose, détecter si celle-ci est conforme au moyen de l'activimètre et procéder à la validation ou la restitution de la dose.

Avantageusement, l'activimètre est contrôlé avec des sources de référence au moyen de tests de constance journalière, de constance, de linéarité, de répétabilité, de reproductibilité et de calibration d'isotope.

De préférence, le procédé comporte l'étape préalable de brancher l'appareil sur secteur et réseau Ethernet en fin de journée pour ensuite, après une certaine durée d'inactivité, effectuer des mises à jour éventuelles puis s'auto-éteindre.

### Brève description des dessins

Les figures 1A à 1D représentent différentes vues en perspective de l'appareil de préparation et d'injection radiopharmaceutique selon un mode de réalisation particulier de l'invention.
Les figures 2A et 2B représentent des vues de l'appareil selon les figures 1A à 1D dans lequel les portes blindées sont ouvertes.
La figure 3 montre schématiquement les composants et le fonctionnement d'une trousse utilisée dans un appareil automatique d'administration de dose radiopharmaceutique selon un mode particulier d'exécution de l'invention.
La figure 4 représente schématiquement un exemple de capture d'écran du menu principal de l'interface utilisateur disponible sur l'écran de contrôle tactile.
La figure 5 représente schématiquement un exemple de capture d'écran d'un sous-menu de l'interface utilisateur relatif aux tests et calibrations de l'appareil.
La figure 6 représente schématiquement un exemple de capture d'écran d'un sous-menu de l'interface utilisateur relatif spécifiquement à l'injection de dose.
La figure 7 représente un exemple de captures d'écran réalistes correspondant respectivement à la figure 4 et à la figure 6.

### Description d'un mode de réalisation préféré de l'invention

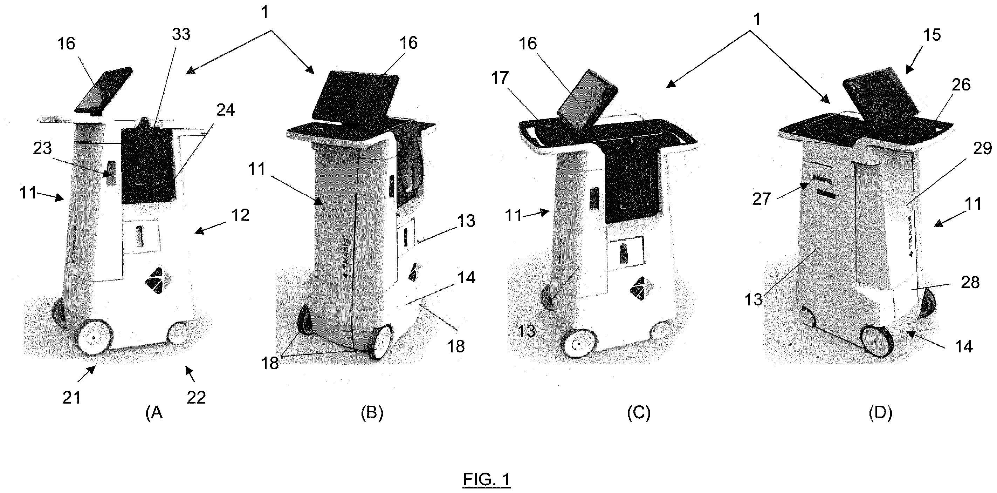

Les figures 1A à 1D montrent différentes vues d'un mode de réalisation de l'automate 1 selon la présente invention.

L'automate 1 comprend une face avant 11, une face arrière 12 et des côtés latéraux 13, qui sont essentiellement verticaux, ainsi qu'une face inférieure 14 et une face supérieure 15, toutes deux essentiellement horizontales, ou le cas échéant légèrement obliques. La face supérieure 15 porte un écran de commande/une interface utilisateur 16 et éventuellement un emplacement de stockage 17, par exemple sous forme d'une auge. L'interface utilisateur 16 susmentionnée est de préférence une interface utilisateur à écran tactile. La face inférieure 14 porte des roulettes 18 qui assurent la mobilité de l'automate, de préférence au nombre de quatre, une roulette se trouvant à chaque coin de la face inférieure 14. De préférence, au moins deux des roulettes 18 sont orientables, par exemple à l'opposé de l'écran 16, tandis que les deux autres roulettes, du côté de l'écran 16, sont fixes.

La partie avant du dispositif est destinée à être placée en face de l'utilisateur (opérateur, personnel infirmier), du côté de l'écran ou de l'interface utilisateur 16, et est donc appelée « côté utilisateur » 21. La partie arrière de l'appareil est destinée à faire face au patient et est donc appelée « côté patient » 22.

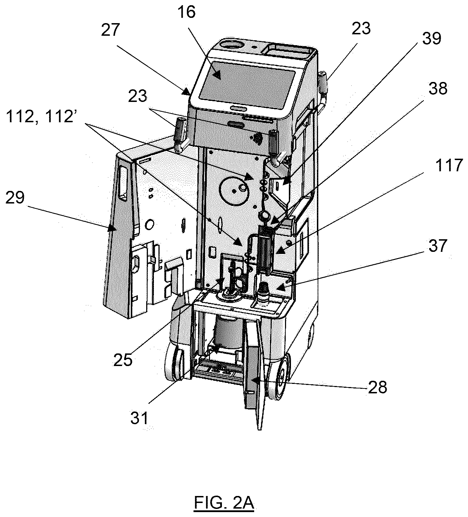

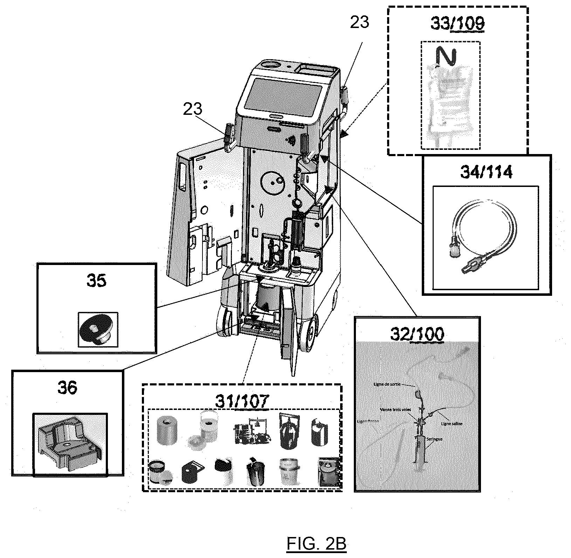

Comme représenté sur les figures 2A et 2B, des poignées 23 sont avantageusement disposées tant sur la face avant 11 que sur la face arrière 12, à l'horizontal et chaque fois au nombre de deux, pour augmenter la maniabilité de l'équipement. Sur chacune de ces poignées 23 se trouve de préférence un dispositif permettant de déverrouiller un dispositif de freinage à main afin de pouvoir faire rouler l'équipement. Ce même dispositif permet aussi d'enclencher une assistance motorisée des roues si l'appareil est équipé de cette option.

De préférence le dispositif de freinage consiste en des roulettes auto-freinées. L'équipement est susceptible d'être déplacé par un opérateur au sein de l'hôpital, en particulier dans le service d'imagerie de tomographie à émission de positrons (TEP), d'une chambre d'injection à l'autre, où des patients individuels sont dans l'attente d'être traités.

La face avant 11 toujours est équipée d'une porte inférieure blindée 28 permettant d'accéder à un flacon blindé 31 contenant le produit radiopharmaceutique et d'une porte supérieure blindée 29 permettant d'accéder aux équipements internes, comme représenté sur la figure 2A (ou 2B) sur laquelle on voit les deux portes respectives inférieure 28 et supérieure 29 ouvertes. Les consommables sont un flacon blindé multidose 31 (généralement acheté par le client, tout comme la poche de saline, voir ci-dessous, en pointillé sur la figure 2B) contenant le produit radiopharmaceutique à injecter au patient, une poche de sérum physiologique 33, une trousse journalière par traceur 32 et un prolongateur (ou ligne patient) 34 pour injection directe au patient.

Des accessoires sont également présents pour l'adaptation du flacon multidose tels que cône de centrage de l'aiguille 35 et adaptateur de flacon blindé 36.

Un premier côté latéral 13, droit, est équipé d'un emplacement 24 pour le positionnement d'une poche de sérum physiologique (saline) 33 généralement suspendue à un crochet et pour la connexion d'une ligne d'injection patient 34. L'autre côté latéral 14, gauche, est équipé d'une imprimante et d'une sortie pour étiquettes imprimées 27.

Une tablette rétractable 26 est disposée de préférence sous et derrière l'écran 16.

L'objectif principal du système est d'administrer une dose intraveineuse de solution radiopharmaceutique à la demande à des fins de diagnostic ou thérapeutiques à partir d'un flacon multidose. En particulier, la fonction remplie par l'appareil et de ses consommables est d'échantillonner, de mesurer et d'administrer automatiquement et en toute sécurité une dose prescrite de produit radiopharmaceutique au patient, lorsque ce dernier est relié à la ligne patient soit par un cathéter, soit le plus souvent à une ligne de perfusion déjà connectée au patient via un cathéter. Le patient est en effet d'abord souvent connecté à une ligne de perfusion avec une poche de saline qui l'hydrate avant injection. Avant de lancer l'injection, l'utilisateur connecte la ligne patient à cette ligne de perfusion la plupart du temps via une rampe de vanne située sur la ligne de perfusion.

Pour l'échantillonnage et l'injection du produit radiopharmaceutique, l'appareil est principalement équipé (voir figure 2B) :
- d'un système de percement motorisé 25 pour percer le flacon contenant le produit radiopharmaceutique en manipulant l'aiguille du consommable principal ;
- d'un pousse-seringue motorisé 37 pour manipuler la seringue du consommable principal, afin de recueillir une dose ultraprécise et de l'injecter ensuite au patient en dilution avec du sérum physiologique.

En outre, pour la mesure de l'activité du produit radiopharmaceutique, l'appareil dispose d'une solution technologique de mesure de l'activité, par exemple grâce à deux radio-détecteurs par exemple de 25 mm (activimètre 117).

Les consommables 31, 32, 33, 34 sont placés dans un emplacement réservé au niveau de l'appareil afin que celui-ci puisse interagir correctement avec eux. Comme l'opérateur doit être radio-protégé, l'équipement 1 est pourvu d'un blindage adéquat. En particulier, les consommables sont placés notamment derrière les deux portes blindées 28, 29. D'autres parties de l'appareil peuvent également être blindées pour ces mêmes raisons de protection. Les moyens d'ouverture et/ou de verrouillage desdites portes sont connus en soi de l'homme de métier.

De plus, selon certaines formes d'exécution non représentées, des accessoires comme un tabouret d'aide au placement/retrait du flacon blindé et de la trousse peuvent être avantageusement utilisés pour améliorer l'ergonomie de ces opérations de début de journée (max. quatre par jour, le reste des opérations se faisant debout). On pourrait également avantageusement utiliser un ascenseur de flacon pour faciliter la manutention du lourd flacon blindé (environ 20kg ; non représenté).

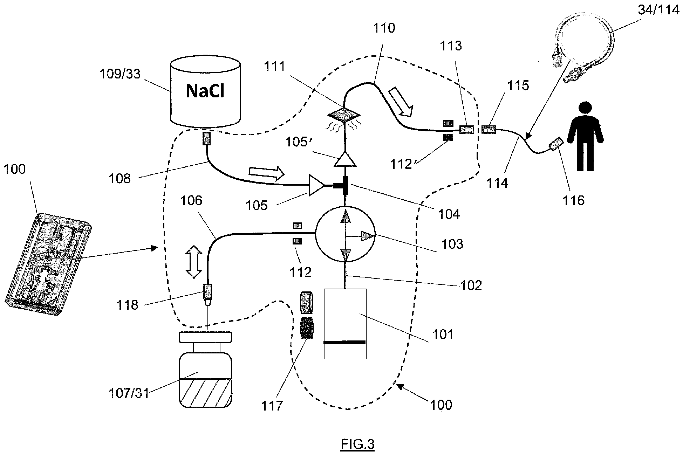

Le schéma de la figure 3 représente, selon un mode d'exécution particulier déjà décrit dans la demande de brevet publiée sous le numéro WO2024061619A1, un exemple de système à trousse 100 (la trousse étant entourée par la ligne pointillée) pour réaliser un dosage au moyen d'une seringue combinée à une vanne trois voies. Le système comprend :
- une trousse 100 comprenant principalement :
   ∘ une seringue 101 dont la contenance nominale est par exemple de 10mL ;
   ∘ une vanne trois voies 103 ;
   ∘ une ligne 106 de prélèvement connectant un flacon de produit radiopharmaceutique 107, via une aiguille 118 perçant le septum du flacon et un tube passant par un détecteur de bulles 112, à la vanne trois voies 103 ;
   ∘ un « T » 104 intégré et reliant la vanne trois voies 103 à une ligne 108 de solution NaCl, à une ligne d'injection 110 et à la seringue 101 ;
   ∘ la ligne 108 connectant par spike le récipient de saline 109 à la ligne d'injection 110 en passant par une vanne anti-retour 105 ;
   ∘ la ligne d'injection 110 comprenant une seconde vanne anti-retour 105', un filtre ventilé 111 et un raccord Luer auto-obturant 113. Cette ligne traverse aussi un détecteur de bulles 112' en amont du raccord auto-obturant 113 ;
   ∘ un filtre hydrophile-hydrophobe 111 pour évacuer l'air et injecter des volumes faibles d'air au patient ;
- le flacon 107 de produit radiopharmaceutique, par exemple de contenance maximale 20mL, rempli avec 10mL (typiquement pour le diagnostic) ou 15/20mL (typiquement pour le thérapeutique) de solution, et muni d'un septum à percer ; le flacon 107 étant connecté à la trousse 100 une fois que l'appareil a déplacé l'aiguille présente dans la trousse au fond du flacon ;
- un récipient contenant de la saline 109 connecté à la trousse via la ligne 108 par une connexion spike ;
- un prolongateur patient 114 connecté en aval de la ligne 110 grâce à un raccord Luer 115 et comprenant à son autre extrémité un autre raccord Luer 116, le connecteur 116 étant apte à se connecter soit à un cathéter, soit à une ligne de transfusion, soit encore à une autre seringue pour la préparation d'une dose dans une seringue externe à la machine en vue de l'administration manuelle de cette dose à un patient ;
- un activimètre 117 se trouvant à hauteur de la seringue 101.

La séquence automatisée des opérations réalisées au moyen de la trousse 100 est de préférence comme suit :
- au préalable, le dispositif remplit toutes les lignes de NaCl pour évacuer l'air et faciliter les prélèvements de doses ; ensuite, le dispositif prélève l'entièreté du flacon pour mesurer l'activité en connaissant de manière précise le volume prélevé ; cette opération permet de déduire la concentration de la solution dans le flacon pour pouvoir prélever des doses précises sur base du volume ;
- une quantité donnée de solution est prélevée du flacon 107 par aspiration au travers la ligne de prélèvement 106 et via la vanne trois voies 103 par la seringue 101 dont le piston est rétracté par un actionneur (non représenté), la voie de prélèvement 106 étant ouverte et la voie d'injection 110 fermée par la vanne trois voies 103 ;
- la concentration de la solution injectable est ajustée en prélevant via la vanne trois voies 103 une quantité appropriée de saline dans le récipient de saline 109, via la ligne 108, la saline étant aspirée dans la seringue 101 ;
- la solution éventuellement diluée est alors injectée via la vanne trois voies 103 par la ligne d'injection 110, la voie de prélèvement 106 étant fermée. Le filtre ventilé 111 empêche le passage de bulles vers le patient et le détecteur 112 le vérifie ;
- la solution est acheminée vers le prolongateur patient 114.

L'homme de métier comprendra que tant les détails de conception et configuration de l'appareil que de la méthode d'administration d'une dose en intra-veineuse à un patient ne sont pas limités à cet exemple particulier de dosage ou à l'utilisation de la trousse spécifique décrite ci-dessus, mais que d'autres formes de réalisation tant pour l'appareil ou pour la trousse que pour l'exécution du dosage sont à la portée de ses connaissances générales.

En résumé, avec une trousse configurée comme indiqué ci-dessus, la seringue aspire une quantité appropriée de solution-mère, l'activité dans la seringue est mesurée avec précision, et corrigée si nécessaire, et son contenu est ensuite poussé par la seringue vers la ligne de sortie. L'activité résiduelle dans la seringue peut encore être récupérée avec une petite quantité de solution saline et poussée vers la ligne de sortie.

Les éléments de base de l'injecteur comprennent un actionneur de seringue de précision et un actionneur pour la vanne à trois voies (non représentés), outre une trousse de tubulures comprenant deux valves anti-retour comme décrit ci-dessus.

D'autres caractéristiques de sécurité ou de commodité d'usage comprennent deux détecteurs de bulles le long des tubes, un filtre, un connecteur Luer auto-obturant, un dispositif de percement du flacon, un dispositif de gestion de l'ouverture sécurisée des portes, un dispositif de positionnement et de stockage d'une source de constance, une imprimante et divers accessoires de stockage (non représentés).

La gestion d'ouverture des portes est effectuée via l'actionneur du percement pour s'assurer que l'aiguille est bien sortie du flacon lorsqu'on ouvre la porte du flacon pour placer ou enlever celui-ci. De plus, l'aiguille se positionne avantageusement dans un logement inaccessible aux doigts de l'utilisateur, ce qui fait qu'il ne peut en aucun cas se blesser avec l'aiguille.

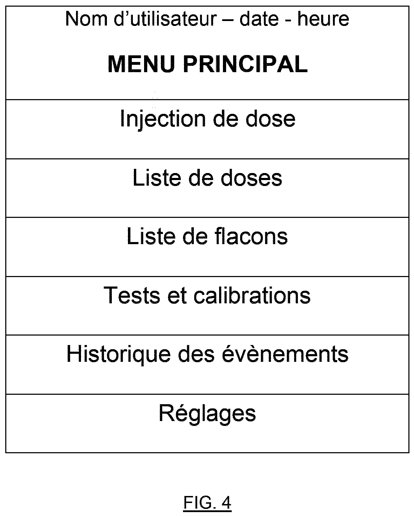

Les figures 4 à 6 montrent, selon une forme d'exécution particulière, les opérations pouvant être effectuées par un opérateur au moyen de l'écran de contrôle du système informatique de l'appareil, au cours d'une journée type d'utilisation.

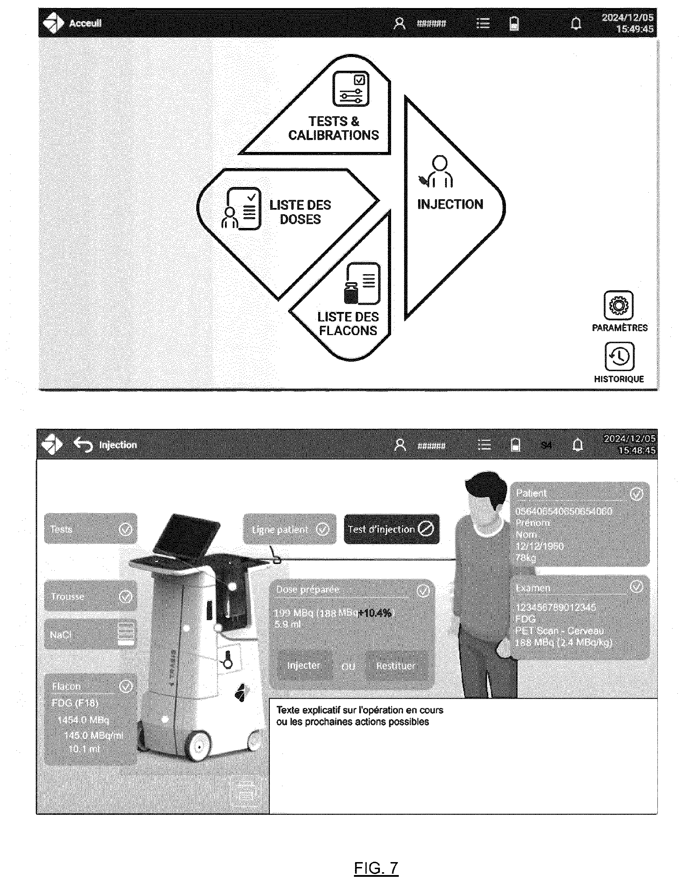

Tout d'abord l'utilisateur va allumer l'appareil qui sera utilisé le cas échéant toute la journée. La figure 4 montre schématiquement un exemple de menu principal auquel il a accès et qui reprend un certain nombre de fonctions : procédure d'injection de dose, choix d'une dose, choix d'un flacon de produit radiopharmaceutique, procédure de tests et calibrations, accès à un historique des évènements et différents réglages (voir aussi figure 7). Ces fonctions correspondent à différents boutons-poussoirs ou zones tactiles connus en soi de l'homme de métier.

Une première opération consiste à tester l'appareil pour s'assurer de sa fiabilité avant usage (cf. bouton « Tests & Calibrations » sur la figure 4). Le test concerne principalement l'activimètre et notamment la constance (activité d'une même source de constance comparée aux mesures des jours précédents), la répétabilité, la reproductibilité, le bruit de fond, etc.

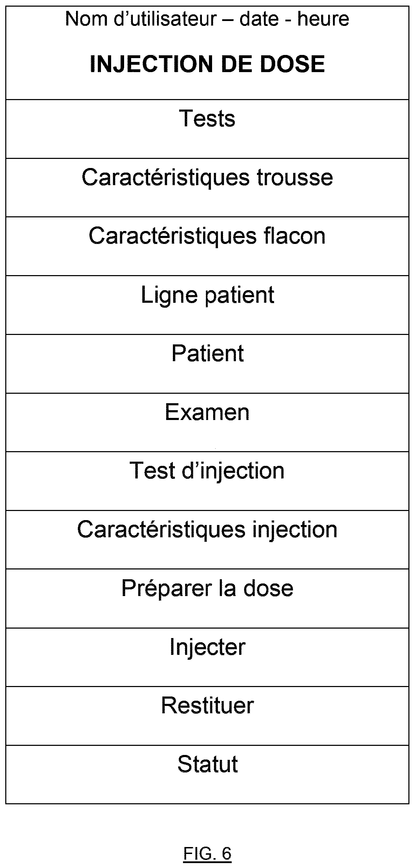

L'utilisateur doit enlever les consommables et le flacon en place pour que ceux-ci n'influencent pas le test. Dans certains cas d'usage, les consommables et flacon sont laissés le soir et enlevés en début de journée ; dans d'autres cas, ils sont enlevés la veille et le matin ; le test de l'activimètre peut être directement effectué. Sur la figure 6, le bouton "Caractéristiques flacon" correspond au bouton "Flacon" de la figure 7 (bas).

Si l'utilisateur clique sur la fonction « Injection de dose », un sous-menu apparaît, représenté schématiquement sur la figure 6. La première opération de la journée est alors, après avoir ouvert la porte inférieure, de retirer le flacon utilisé la veille en cliquant sur « Caractéristiques flacon » ou « Flacon », ce qui permet de déverrouiller et d'entrouvrir la porte inférieure et ensuite, après avoir ouvert la porte supérieure, de retirer la trousse, la ligne patient et la poche de saline utilisés la veille en ayant préalablement cliqué sur « Caractéristiques trousse » ou « Trousse », ce qui permet de déverrouiller et d'entrouvrir la porte supérieure. Optionnellement le test journalier de vérification, notamment de l'activimètre, qui est obligatoire, peut aussi être réalisé après chaque changement de trousse en cliquant sur « Tests » et en incorporant dans le compartiment de l'activimètre une source de référence. Après le test, l'opérateur peut soit écarter la source de référence de l'activimètre tout en restant dans l'appareil (par exemple dans un logement blindé), soit retirer purement et simplement la source de référence de l'équipement. Ensuite de nouveaux consommables (poche de saline, trousse et ligne patient) vont être placés en utilisant les fonctions comme ci-dessus, à savoir d'abord le placement de la trousse, de la poche de saline et de la ligne patient (« Caractéristiques trousse ») et ensuite le placement du flacon (« Caractéristiques flacon »). Une fois la porte supérieure fermée, la mise en place de la poche de saline va permettre le remplissage de toutes les tubulures.

Un capteur de force est en outre prévu pour détecter si on a oublié de mettre la poche de saline ou pour détecter le bon déroulement des mouvements de fluides dans les consommables (ex. : détection d'obstruction/de tubes croqués, détection de fin de remplissage de saline grâce à la montée en pression lorsque la saline atteint le filtre hydrophobe en bout de ligne patient, détection de ligne patient connectée - si elle ne l'est pas, la saline arrive en butée sur l'auto-obturant de la ligne d'injection de la trousse, etc.). Cela permet alors de stopper le remplissage de saline pour éviter de mettre le circuit en surpression et de garantir un bon remplissage de la trousse et la ligne patient avec de la saline et enfin qu'il y ait des quantités négligeables d'air dans les consommables (non représenté).

Un voyant « Ligne patient » va indiquer si la ligne patient a été détectée. La trousse et la ligne patient ont toutes deux leur propre étiquette RFtag pour permettre une lecteur automatique des données de ces deux consommables (N° de lot pour traçabilité, volume intérieur pour le bon remplissage de saline, etc.) après que l'utilisateur ait placé ces étiquettes dans leur logement sur le dispositif. Ce sont ces RFtag et les détecteurs de bulles qui permettent de détecter la présence des consommables. Avantageusement, les boutons sont prévus pour changer de couleur, ce qui indique à l'utilisateur que les consommables sont bien placés.

Ensuite, l'opérateur sélectionne un patient ainsi que la dose prescrite (« Patient », « Examen »). Avant la préparation de dose, on prévoit une opération facultative qui s'intitule «Test d'injection» et qui permet d'injecter une dose patient équivalente à celle prescrite mais sans activité, et donc avec uniquement de la saline en utilisant le débit prescrit. Cela a un intérêt pour des patients avec un terrain veineux fragile, pour s'assurer qu'il n'y aura pas d'extravasation et donc pour ne pas que la solution radioactive s'écoule en dehors de veines (cathéter mal mis, veine qui éclate, etc.). Dans la plupart des cas, ce test n'est pas nécessaire ou il est déjà réalisé manuellement pas l'infirmier(-ère) lors du placement du cathéter et de la ligne de perfusion.

Suite à cela, vient la préparation de dose (« Préparer la dose »). L'utilisation du pousse-seringue permet de préparer la dose de manière précise. L'activimètre va afficher la valeur précise de la dose préparée. C'est ensuite l'utilisateur qui va pouvoir comparer la dose demandée par rapport à la dose réellement préparée. C'est également lui qui va décider ou non d'injecter la dose préparée en appuyant sur le bouton «Injecter». S'il ne souhaite pas procéder à l'injection, il peut choisir de «Restituer» la dose soit dans le flacon initial, soit dans un flacon blindé externe, soit encore dans une seringue blindée externe. Il existe des garde-fous automatisés qui empêchent l'injection de dose préparée si celle-ci est x% plus élevée ou y% plus faible que celle demandée. Le dispositif autorise un contournement de ces garde-fous pour des utilisateurs avertis grâce à une gestion des utilisateurs dans le logiciel avec gestion d'accès sécurisé par mot de passe.

En résumé, l'activimètre permet donc à l'utilisateur de détecter si la dose est correcte ou non. Dans le cas contraire, il « rejette » la dose (« Restituer »). Si la dose est correcte, l'utilisateur connecte le patient à la ligne et lance l'injection (« Injecter »).

Au cours de la journée, les patients peuvent se succéder et pour chaque nouveau patient, on va remettre une nouvelle ligne et répéter les étapes précédentes, sauf éventuellement les premières (remplacement du flacon). Au placement de la nouvelle ligne patient, le dispositif remplit automatiquement la ligne de saline à partir de la poche de NaCl déjà en place. Une poche de saline tient environ une journée voire plus. Cette poche est donc changée préférentiellement en début de journée avec la trousse et est conservée toute la journée pour autant que la trousse ne soit pas changée. Il est conseillé qu'à chaque changement de trousse, la poche de saline soit également changée.

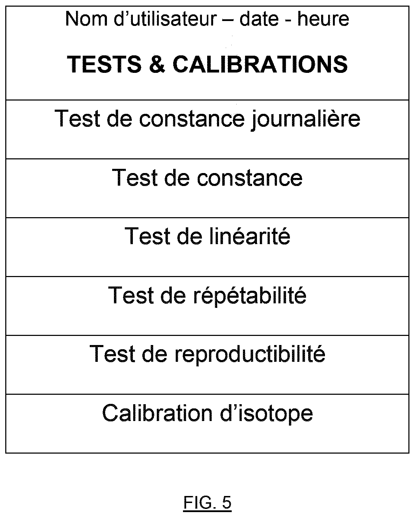

La calibration de l'appareil est gérée via le sous-menu « Test & Calibrations » (Figure 5). Différents tests sont ainsi prévus pour le contrôle de l'activimètre avec des sources de référence.

### Liste des symboles de référence

- 1: appareil mobile pour injection
- 11: face avant
- 12: face arrière
- 13: côté latéral
- 14: face inférieure
- 15: face supérieure
- 16: interface utilisateur
- 17: emplacement de stockage
- 18: roulette
- 21: côté utilisateur
- 22: côté patient
- 23: poignée
- 24: emplacement saline et connexion ligne patient
- 25: système de percement
- 26: étagère rétractable
- 27: sortie imprimante
- 28: porte inférieure blindée (flacon)
- 29: porte supérieure blindée (trousse)
- 31: flacon blindé de produit radiopharmaceutique
- 32: trousse de tubulures
- 33: poche de saline
- 34: ligne patient
- 35: cône de centrage aiguille
- 36: adaptateur de flacon blindé
- 37: pousse-seringue
- 38: actionneur rotatif de vanne
- 39: lecteur RFtag
- 100: trousse radiopharmaceutique
- 101: seringue
- 102: ligne de connexion vanne-seringue
- 103: vanne trois-voies
- 104: tube en « T »
- 105, 105': vanne anti-retour
- 106: ligne de prélèvement
- 107: flacon de produit radiopharmaceutique concentré
- 108: ligne de connexion de saline
- 109: flacon de saline
- 110: ligne d'injection
- 111: filtre ventilé
- 112, 112': détecteur de bulles
- 113: Luer auto-obturant
- 114: prolongateur patient
- 115: raccord Luer machine
- 116: raccord Luer patient
- 117: activimètre
- 118: aiguille de percement

## Revendications

1. Un appareil mobile (1) pour la préparation et l'injection ou la perfusion automatique à la demande d'une dose de produit radiopharmaceutique à une pluralité de patients, comprenant une pluralité de compartiments au moins partiellement blindés :
- un premier compartiment pour accueillir une trousse de tubulures (32, 100) permettant de préparer avec précision via plusieurs chemins de fluide une dose de produit radiopharmaceutique dilué avec de la saline (33, 109) directement injectable à un patient ;
- un deuxième compartiment pour accueillir un flacon multidose (31, 107) de produit radiopharmaceutique ;
- un troisième compartiment pour accueillir un conteneur de saline (33, 109) et la connexion à une ligne patient (34, 114) ;
ladite trousse (32, 100), le conteneur de saline (33, 109) et ledit flacon blindé (31) de produit radiopharmaceutique étant des consommables configurés pour être remplacés après traitement d'un ou plusieurs patients ;
ladite trousse (32, 100) comprenant une seringue commandée (101) pour prélever le produit radiopharmaceutique après percement au moyen d'un système motorisé d'un septum du flacon de produit radiopharmaceutique (31, 107) et une vanne trois-voies (103) qui permet successivement de prélever le produit radiopharmaceutique dans la seringue (101), de le mélanger à une quantité de saline (33, 109) et de l'injecter à un patient selon trois chemins de fluide successifs (106, 108, 110) ; ainsi que
- des moyens de préparation et d'administration de fluide comprenant un actionneur de précision de la seringue commandée (101) et un actionneur de la vanne trois-voies (103) ;
- un activimètre (117) disposé à proximité de la seringue commandée (101) pour valider avant injection la dose préparée ;
- un contrôleur en communication avec les moyens de préparation et d'administration de fluide configuré pour recevoir d'un utilisateur des informations de préparation de dose pour un patient et pour communiquer des instructions correspondantes aux moyens de préparation et d'administration de fluide ;
- une interface de communication (16) entre ledit contrôleur et un utilisateur, capable, via un menu d'instructions, de permettre à l'utilisateur de lancer une procédure de préparation et d'injection de dose, des tests, des calibrations et un accès à des données en mémoire, par exemple relatives à l'historique des injections de dose ; et
- des moyens de stockage et d'impression des données.

2. L'appareil mobile selon la revendication 1, **caractérisé en ce que** l'appareil est un automate mobile, par exemple un chariot mobile (1) monté sur une pluralité de roulettes (18), déplaçable et guidable au moyen de poignées (23) disposées au moins sur une face avant (11) et muni d'un système de freinage, de préférence grâce à des roulettes autofreinées, ou d'une assistance motorisée des roulettes.

3. L'appareil mobile selon la revendication 1, **caractérisé en ce que** lesdites roulettes (18) sont au nombre de quatre, à raison d'une à chaque coin de l'appareil, deux roulettes fixes au niveau d'une face avant (11), côté utilisateur, et deux roulettes orientables au niveau d'une face arrière (12), côté patient, de l'appareil.

4. L'appareil mobile selon la revendication 1, **caractérisé en ce que** l'interface de communication (16) est une interface utilisateur à écran tactile et disposée sur une surface supérieure (15) de l'appareil.

5. L'appareil mobile selon la revendication 1, **caractérisé en ce que** l'appareil est muni sur une face essentiellement verticale (12), côté patient, d'un premier accès par une première porte blindée (29) au premier compartiment destiné à contenir la trousse de tubulures (32, 100) et d'un second accès par une seconde porte blindée (28) au deuxième compartiment destiné à contenir le flacon multidose (31, 107) de produit radiopharmaceutique.

6. L'appareil mobile selon la revendication 5, **caractérisé en ce que** le déverrouillage et/ou l'entrebaillement des première (29) et seconde (28) portes blindées est commandable au moins une fois par jour par l'opérateur à partir de l'interface de communication (15).

7. L'appareil mobile selon la revendication 1, **caractérisé en ce qu'**il comprend un capteur de force pour détecter la présence d'une poche de saline (33, 109) et le bon déroulement des mouvements de fluide dans les consommables dont le flacon multidose (31, 107), la trousse (32, 100), le conteneur de saline (33, 109) et la ligne patient (34, 114).

8. L'appareil mobile selon la revendication 1 ou 5, **caractérisé en ce qu'**il comporte pour l'échantillonnage et l'injection du produit radiopharmaceutique :
- un actionneur de percement motorisé (25) pour percer le flacon contenant le produit radiopharmaceutique en manipulant l'aiguille de la trousse (32) ;
- un pousse-seringue motorisé (37) pour manipuler la seringue de la trousse (32), en vue de recueillir une dose ultra-précise et de l'injecter ensuite au patient en dilution avec du sérum physiologique.

9. L'appareil mobile selon la revendication 8, **caractérisé en ce qu'**il comprend des moyens pour une gestion d'ouverture des portes (28, 29) effectuée via l'actionneur de percement (25) pour s'assurer que l'aiguille est bien sortie du flacon lorsqu'on ouvre la porte du flacon (28) et des moyens pour assurer que l'aiguille se positionne dans un logement inaccessible aux doigts de l'utilisateur.

10. Un procédé de mise en oeuvre d'un appareil mobile pour la préparation de doses de produit radiopharmaceutique destinées à l'injection ou la perfusion automatique à la demande à des patients, généralement répartie sur une journée, selon l'une quelconque des revendications précédentes, **caractérisé par** les étapes suivantes :
- mettre l'appareil (1) sous tension au début de la journée ;
- préparer l'appareil (1) pour la journée, à savoir :
∘ enlever les consommables dont le flacon multidose (31, 107), la trousse (32, 100), le conteneur de saline (33, 109) et la ligne patient (34, 114), s'ils sont toujours présents depuis la veille, après ouverture de la première porte blindée (29) et de la seconde porte blindée (28), le flacon (31, 107) étant toujours enlevé avant la trousse (32, 100) pour réduire les risques d'exposition radioactive ;
∘ effectuer le test journalier de vérification, en particulier de l'activimètre (117) en utilisant une source de référence ;
∘ mettre en place dans l'appareil via l'ouverture des portes précitées (28, 29) de nouveaux consommables, à savoir au moins respectivement un flacon multidose (31, 107) de produit radiopharmaceutique, à destination de plusieurs patients, ainsi qu'une trousse de tubulures (32, 100) et un conteneur de saline (33, 109) ;
∘ mettre en place une ligne patient (34, 114) pour chaque nouveau patient ;
- démarrer la procédure automatique de préparation de dose ;
- laisser les tubulures se remplir par la poche de saline et mesurer l'activité dans le flacon en connaissant de manière précise le volume prélevé ;
- détecter la ligne patient (34, 114) ;
- sélectionner, corriger et/ou encoder sur l'interface de communication (16) un patient déterminé et une dose prescrite ;
- effectuer la préparation de la dose, détecter si celle-ci est conforme au moyen de l'activimètre (117) et procéder à la validation ou la restitution de la dose.

11. Le procédé de mise en oeuvre de l'appareil selon la revendication précédente, **caractérisé en ce que** l'activimètre (117) est contrôlé avec des sources de référence au moyen de tests de constance journalière, de constance, de linéarité, de répétabilité, de reproductibilité et de calibration d'isotope.

12. Le procédé de mise en oeuvre de l'appareil selon la revendication 10, **caractérisé en ce qu'**il comporte l'étape préalable de brancher l'appareil sur secteur et réseau Ethernet en fin de journée pour ensuite, après une certaine durée d'inactivité, effectuer des mises à jour éventuelles puis s'auto-éteindre.
